# EUROPEAN PATENT APPLICATION

(11) **EP 1 459 714 A1**
(43) Date of publication of application: **22.09.2004**
(21) Application number: 02793452.0
(22) Date of filing: 27.12.2002
(51) Int. Cl.: A61F 7/00

(54) **WHOLE-BODY THERMOTHERAPY METHOD AND DEVICE**

(30) Priority: 28.12.2001 JP 2001399543
(71) Applicant: Sakamoto, Noriyasu, Tokyo 179-0076 (JP)
(72) Inventor: Sakamoto, Noriyasu, Tokyo 179-0076 (JP)
(74) Representative: Staudt, Hans-Peter, Dipl.-Ing.
(86) International application number: PCT/JP2002/013834
(87) International publication number: WO 2003/057098

(57) **Abstract**

A whole-body thermotherapy is conducted by letting a patient take a warm bath while a measure for minimizing a brain stress is taken and an internal temperature is measured. The measure taken to prevent the stress includes the method of using blue, light blue or any other colored water as a warm water source for bathing, the method of applying a chromatic light to the eyes of a patient, the method of letting a patient hear a sound, the method of stimulating meridians or vital spots, the method of cooling the head, and the method of attaching a bioelectric current regulating element consisting of at least two kinds of metal or two kinds of gem to a patient or to the patient-touching portion of a bed to relieve a brain stress by the actions of metal ions and magnetic fields. Accordingly, a whole-body thermotherapy can be practiced while pain of and load on a patient are alleviated as much as possible.

## Description

### TECHNICAL FIELD

The present invention relates to a whole-body hyperthermia (hereinafter referred to as "WBH") for cancer treatment and an apparatus therefor.

### BACKGROUND ART

Ever since it became evident in medical circles in late 1970's that cancer cells die out when a certain temperature is reached, WBH has become an area of vigorous research. Taking advantage of cancer cell properties listed below, WBH is extremely simple and easy to understand in its principle

Firstly, cancer cells are characterized in that they are more heat sensitive than normal cells, i.e., they are vulnerable to heat. Temperatures of 42 to 43°C, not problematic for normal cells, have been demonstrated to be a fatal environment for cancer cells.

Secondly, cancer cells are characterized in that they are lower in blood flow than normal cells. That is, it can also be said that cooling effect of cells, normally obtained from increased blood flow, is nonexistent, resulting in heat being readily confined within such cells.

In other words, thermal cancer therapy is designed to heat cancer cells to a certain constant temperature not affecting normal cells, thus promoting cell deaths in affected area.

WBH is allegedly effective for the reasons given below.
1. Since cancer cells are vulnerable to heat, tumor cells die out at an increasingly fast pace with more heat.
2. While cancer cell blood vessels expand when heated, they begin to contract at temperatures of 41 to 42°C, resulting in an ischemic condition in cancer tissues. Although blood vessels expand first and then begin to contract even in normal cells (tissues), cancer tissues start their contraction at lower temperatures, leading to an ischemic condition.
3. While cancer may be caused by virus, virus is also vulnerable to heat. Virus, most comfortable at 37°C, becomes less active at 38°C and beyond. As described above, cancer stops growing or necrosis takes place.

Optimum treatment temperature and time according to cancer type are as shown in Table 1, whereas optimum treatment temperature and time for HIV virus is as shown in Table 2.

It is to be noted that optimum treatment temperature and time are known to vary depending on cancer type classified pathologically (anti-cancer drug/radiation treatment is generally known to differ in effect according to organ and individual), irrespective of organ type.

Cancers vary in thermal sensitivity from one type to another. A comparative experiment (thermal sensitivity experiment), using 50,000 endometrial adenocarcinoma cells and 50, 000 squamous cell carcinoma cells stabilized in 2cc of culture solution in laboratory dishes under a temperature condition of 37°C for 24 hours, produced experimental data of the National Institute of Infectious Diseases as follows:
- Endometrial adenocarcinoma 41.0°C (died out after three hours of treatment)
- Squamous cell carcinoma 42.0°C (died out after three hours of treatment)

Thus, WBH has been substantiated to be effective in cancer treatment, leading today to development of various WBHs.

Typical among them is extracorporeal circulation hyperthermia. Extracorporeal circulation hyperthermia consists of making percutaneous punctures in the right or left femoral artery and vein and inserting a French-sized canula by the Seldinger method under whole-body anesthesia, extracting blood from the femoral artery, heating blood from 37°C to 45°C with a heat exchanger and returning blood to the femoral vein.

However, this method, by which thick needles are punctured into the femoral artery and vein, is problem-prone and requires the patient, for example, to remain on bed for three to four hours after treatment.

As a replacement of problematic extracorporeal circulation hyperthermia as described above, various forms of WBH such as WBHs using far and infrared rays have been developed.

In WBH using far or near infrared ray, the patient is laid down in a space called chamber, with far or near infrared ray irradiated to the body surface, thus heating the inside of the body via the skin.

With this method, however, it was impossible to simultaneously irradiate light from all directions - from front and back, both sides and above and below the body for heating.

In order for the largest area to be irradiated, the patient must stand up and be irradiated from front and back, requiring the patient to remain standing up for two to three hours. It is, however, difficult for a languishing patient to maintain a standing posture for long hours. And, even in this posture, the patient's soles cannot be irradiated.

For this reason, the patient is normally irradiated while lying down, but the areas that can be irradiated are further limited in a lying down posture.

Conventional hyperthermia using far infrared ray, etc. require temperatures from 60°C to 78°C, making the therapy unbearable unless the patient is put under constant anesthesia. Besides, there was a concern over adverse impact of anesthesia to the cranial nervous system.

According to the present invention, however, it is possible to raise the internal body temperature beyond 40°C using a warm source at 40°C to 45°C, thus allowing administration of hyperthermia with a restricted supply of anesthesia gas while at the same time supplying a sufficient amount of oxygen. This makes it therefore possible to prevent adverse impact to brain cells to the extent possible.

### DISCLOSURE OF THE INVENTION

The WBH of the present invention is characterized in that patients are subjected to warm bathing while at the same time administering treatment to minimize brain stress and measuring the internal body temperature. In warm bath, temperature drop of water or other warm source can be prevented by covering the bathtub and leaving only the head of the patient outside the cover or putting the whole-body of the patient including his or her head inside the bathtub and supplying oxygen and anesthesia gas with a pipe running through the cover.

Means listed below are possible as the treatment means for minimizing brain stress. These means may be employed alone or in combination.

### (1) Use colored water such as light blue or blue as a warm source of warm bath

Findings from an experiment - experiment in which subjects were shown red and light blue images for five minutes and change in body temperature was examined before and after they were shown images - showed that the body temperature increased in many subjects when they saw the red image while the body temperature dropped in many subjects when they saw the light blue image (Table 3). This leads to an assumption that using red-colored water in the bathtub allows the body temperature to increase even when the water temperature is relatively low, whereas using light-blue- or blue-colored water calms down brain cells, alleviating stress.

For this reason, results of Table 3 are applicable to warm bathing as follows.

On one hand, warm bathing can be employed for patients highly resistant to stress to the body resulting from increased skin temperature.

Such patients can continue warm bathing for long hours with the warm source temperature remaining high. For this reason, red color effect from red-colored water or other warm source together with heat from the warm source maintained at a high temperature promotes temperature increase inside the body.

On the other hand, warm bathing can be used in two ways for patients with low stress resistance.

Firstly, warm source is colored red, thus promoting temperature increase inside the body through red color effect while maintaining the warm source temperature relatively low. The experiment confirmed that warm source, even at 41 degrees, is as effective, when colored red, as that at 42 degrees.

Secondly, warm source is colored light blue or blue, thus alleviating stress to the internal body even when the warm source temperature is relatively high. The experiment confirmed that blue-colored warm water alleviates stress more than colorless warm water both in patients' heart rate and breathing data and their self-declaration.

It is to be noted that, in addition to light blue and blue, use of other colors are also probably preferred such as a mixture of blue and green, a color effective in improving amnesia, green effective for cardiac disorder, indigo effective for headache and red purple effective for high blood pressure (Table 4).

### (2) Warm bathing while shining colored light on the eyes

It is possible to obtain the same effect by shining colored light (including image) on the eyes rather than coloring the warm source.

### (3) Warm bathing while allowing the patient to listen to music

From the fact that amnesia improves by allowing patients complaining of amnesia to continuously listen to "deep breathing-in sound" and "deep breathing-out sound" during deep breathing or music (e.g., Mozart) (Table 5), it can be assumed that stress is alleviated by breathing sounds and music.

From the fact that amnesia improves further by continuously playing breathing sounds or music together with images, it can be assumed that brain stress is further resolved by playing sounds and coloring the warm source of warm bathing in light-blue or other color or shining colored light.

### (4) Warm bathing while stimulating shiatsu or acupuncture spots

Stimulating shiatsu or acupuncture spots of the areas outside the warm source such as ears, head and hand fingers provides an easing effect, replacing anesthesia gas and resolving whole-body or brain stress. For stimulation, techniques such as acupuncture/moxa, low frequency and laser irradiation (medium and high frequencies included) can be employed as appropriate.

### (5) Warm bathing while cooling the head

Cooling the head reduces the temperature of blood flowing into brain cells and calms down these cells, resolving brain stress.

### (6) Attaching a biological current adjustor, consisting of two or more metals or jewels of different kinds, to the patient or to a portion of the bed coming in contact with the patient, resolving brain stress by the action of metal ions and magnetic field (refer to Tables 6 and 7).

It is preferred that not only water, seawater, distilled water, hot spring water, aqueous vitamin solution, aqueous aroma solution, paraffin water, chitin/chitosan solution, vinegar, acetic acid, liquors, alcohols (those that may be diluted with vinegar, acetic acid, liquor, alcohol or water) but also those having a high specific heat such as mud (mud mixed with moisture) and sawdust mixed with moisture (wood dust), higher than water in specific heat, be used as a warm source for warm bathing.

The seawater provides, thanks to its salt content, an effect of raising the body temperature higher than its actual temperature as with red-colored water, and a similar effect can be expected of hot spring water. Aqueous aroma solution, on the other hand, provides a stress alleviating effect.

Specific heat is the quantity of heat required to raise the temperature of the substance. Therefore, a substance with a higher specific heat has more heat than another substance with a lower specific heat if they are at the same temperature. Consequently, a substance with a high specific heat has the property of being difficult to be heated but difficult to cooled, surrounding the whole-body with more quantity of heat and allowing warm heat to penetrate deeply and uniformly into the body.

Water is approximately 27-fold higher than air in specific heat, whereas mud is greater than water in specific heat.

Use of a substance with a high specific heat ensures deep and uniform penetration of warm heat into the whole-body, resulting in cell deaths of even extremely small cancers nearing birth, previously not possible to discover, irrespective of size and location.

The invention according to claim 4 is characterized in that the patient drinks, during warm bathing, one or more of water, aqueous trace element solution and aqueous solution in which trace elements are copied to potassium iodide water.

It is preferred that water free of carcinogenic substances such as trihalide methane and solvents likely to adversely affect the body, deep sea water, mineral water, π water (trade name), alkali-ion water, electrolyzed water (acid water, reduced water) and other functional water be used as the water.

The trace elements are those reported as deficient by international medical circles, namely, iron, zinc, manganese, selenium, iodine, chrome and cobalt as a constituent ingredient of vitamin B21. Among others, a combination of iron and iodine is preferred.

Patient's water consumption makes it possible to cool down the head and resolve brain stress.

Patient's water consumption results in cancer cell and virus deaths through hyperthermia and, at the same time, allows improvement of the patient's physical constitution.

Consumption of aqueous trace element solution or aqueous solution, in which trace elements are copied to potassium iodide water, sends the trace elements into cancer cells, destroying these cells. Table 8 is clinical trial data in which a colloidal solution prepared by mixing iron and iodine, trace elements, into aqueous potassium iodide solution was consumed by terminal cancer patients. According to this data, efficacy was observed in 89.6% of the patients.

A comparison was made in respect of recovery time after hyperthermia, on 23 subjects, between hyperthermia with and without water consumption. Findings showed that 22 subjects recovered within 15 minutes with water consumption and that recovery took 30 minutes or more for 19 subjects without water consumption.

An experiment conducted on the same subjects in respect of the head temperature showed the head temperature was 0.3 degrees or more lower with water consumption than without water consumption for 28 subjects.

The acid water refers to water gathering on the positive pole in an electrolytic layer screened off by diaphragm and is approximately pH4.5 to 5.5.

Acid water, not often used as potable water, is well known in medical circles to be outstandingly effective against abdominal pain, diarrhea, diabetic gangrene, burn, frostbite and so on by the action of conjugate proton "H⁺pluse⁻", if consumed under an emergency condition.

Acid water of pH3.0 or less for commercial use, on the other hand, is powerfully disinfectant and employed for MRSA fungi, athlete's foot fungi, atopic dermatitis and hemorrhoids disorder.

The reduced water refers to water gathering on the negative pole in the electrolytic layer and is approximately pH8.5 to 10.5. Dissolved oxygen is 5 to 6ppm, with calcium, magnesium and sodium ions contained in abundance.

Reduced water is high in thermal conductivity and has a powerful swelling function, dissolving nutritive substances well, ensuring good absorption into the body and converting nutritive substances to energy without waste.

The following is supplementary information regarding the water.

It is a known fact that 60 to 75% (weight) of the human body is water, and the amount of water (weight) contained in each organ is roughly as follows. That is, 83.0% in blood, 82.7% in kidneys, 79.2% in heart, 79.0% in lungs, 75.8% in spleen, 75.6% in muscles, 74.8% in brain, 74.5% in intestines, 72.0% in skin, 68.3% in liver, 22.0% in skeleton and 10.0% in adipose tissue.

In adult's digestive tract, eight to nine liters of water are normally constantly stored, whereas about 144 liters of water are circulated in kidneys a day.

Water functions in two ways in digestive tract, i.e., solubility and dispersibility.

Solubility refers to the role of a substance to be well mixed into a liquid so as to ensure dissolution of a digested matter, whereas dispersibility denotes the role to bring about a reaction to digest water and food, that is, transform fat and protein into a mayonnaise-like state through cisele reaction so as to facilitate absorption into intestine.

A large amount of energy is consumed in cells and mucous and biological membranes to maintain the human body in good health and to transfer and digest heat energy in hyperthermia. At this time, the aforementioned functions of water (solubility and dispersibility) are essential to ensure that abundant energy is properly consumed. Therefore, water consumption during hyperthermia allows water to readily deliver its functions.

The following is supplementary information regarding the functional water.

In "Pharmaceutical Affaire No.763", a notification was issued by the Director of the Pharmaceutical Affaires Bureau of the Health and Welfare Ministry to the prefectural governors nationwide, approving the effect and efficacy of electrolyzed water (negative pole water and positive pole water), and the Health and Welfare Ministry established in 1993 the Functional Water Foundation in Shibuya-ward, Tokyo, to transmit correct information about electrolyzed water.

The pamphlet of the Foundation states in an explanation of functional water that: "It is generally known that interaction between various ion types of water as a solvent and solute or interaction between water and ion types in aqueous solution system changes considerably by external force. In our foundation, aqueous solutions, that can have sophisticated applications such as those primarily applicable for medical use, among those produced by applying external force to the aqueous solution system and performing physicochemical treatment are defined as functional water."

The inventions according to claims 5 and 6 relate to a apparatus for carrying out the inventions according to claims 1 to 4.

The invention according to claim 5 is characterized in that there are provided means for measuring the bathtub temperature and the internal head temperature of the patient, means for measuring the internal body temperature of the patient and means for resolving brain stress of the patient.

The invention according to claim 6 is, as means for resolving brain stress, one or a combination of two or more of (1) means for shining colored light on the patient's eyes, (2) means for allowing the patient to listen to sounds, (3) means for stimulating shiatsu or acupuncture spots, (4) means for cooling the head and (5) biological current adjustor consisting of two or more metals or jewels of different kinds.

While among the means for shining colored light on the patient's eyes are a light source or monitor screen emitting light through a colored sheet, colored glasses may be worn by the patient.

While a speaker is acceptable as the means for allowing the patient to listen to sounds, headphones are preferred because sounds enter the ears directly while shutting out external sounds.

Among possible options as the means for stimulating shiatsu or acupuncture spots are attaching a small-sized vibrator to the patient or fitting it to the hat worn by the patient. Alternatively, a construction may also be used by which acupuncture/moxa, low-frequency irradiation or laser irradiation is performed.

Among possible options as the means for cooling the head are attaching ice or a chemical substance having a cooling effect (e.g., "ICE-NON": trade name) to the head, and cold water shower.

As for the means for measuring the internal head temperature, means are used that are designed to insert a tube through the nose to measure the internal head temperature or designed to externally measure the internal head temperature (e.g., THERMO's Core Thermometer: trade name).

As the means for measuring the internal body temperature, means are used in which a sensor is inserted through the anus to measure the temperature of the rectum. Incidentally, the rectum temperature is said to be close to the body core temperature.

In the present invention, the patient is subjected to warm bathing while resolving brain stress, providing a high warm bathing effect. It becomes possible, by subjecting the patient to warm bathing while measuring the internal body temperature, to ensure an optimum treatment temperature during warm bathing. Use of mud or other substance with a high specific heat as a warm source allows the body to be heated to the core without undue effort, making it possible to treat cancer cells existing deep inside the body.

Moreover, it is possible in the present invention to raise the internal body temperature beyond 40°C using a warm source at 40°C to 45°C, thus allowing administration of hyperthermia with a restricted supply of anesthesia gas while at the same time supplying a sufficient amount of oxygen. This makes it therefore possible to prevent adverse impact to brain cells to the extent possible.

It is to be noted that, in addition to cancers, the present invention is likely effective for treatment of leukemia, HIV, C-type hepatitis, Parkinson's disease, collagen disease, malaria, sleeping sickness and elephantiasis. It is to be noted that hyperthermia has been confirmed to be effective in treating protozoan diseases such as malaria, sleeping sickness and elephantiasis.

### BRIEF DESCRIPTION OF THE DRAWING

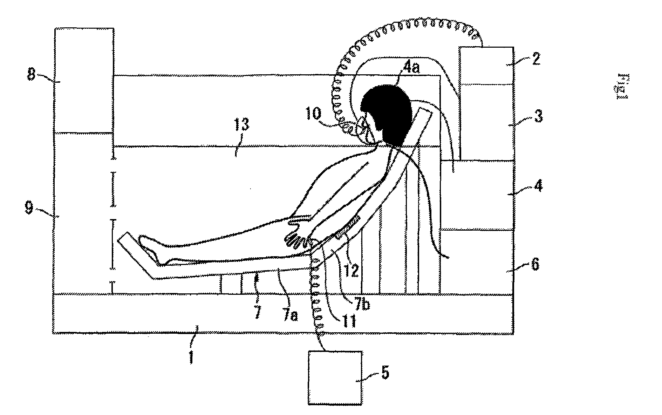

Fig. 1 is a sectional view showing an embodiment of the present invention.

### Explanation of Reference Numerals

1 Bathtub
2 Thermometer
3 Anesthesia apparatus
4 Head cooling apparatus
5 Thermometer
6 Instillation apparatus
7 Bed
8 Level sensor
9 Heating apparatus
10 Tube
11 Sensor
12 Magnetic field inducer
13 Warm source

### BEST MODE FOR CARRYING OUT THE INVENTION

In the figure, a whole-body hyperthermia apparatus of the present invention is configured with a bathtub 1, a thermometer 2 for measuring the internal head temperature, an anesthesia apparatus 3, a head cooling apparatus 4, a thermometer 5 for measuring the internal body temperature and an instillation apparatus 6, with a bed 7 disposed in the bathtub 1 and a level sensor 8 and a heating apparatus 9 also provided therein.

The thermometer for measuring the internal head temperature, intended to grasp the magnitude of brain stress as a temperature change, measures the internal head temperature with a tube 10 inserted from the nose and displays the measured value on a control panel (not shown).

The anesthesia apparatus 3 is designed to administer gas anesthesia. Anesthesia is administered by injection before the patient enters the bathtub, and then gas anesthesia is administered once the patient is in the bathtub. Anesthesia relaxes the patient's muscles and the patient, making it easy to obtain a hyperthermia effect.

The head cooling apparatus 4 is configured by providing shower nozzles for cool water shower or cool air injection nozzles in a fitting portion 4a in the form of a hat. In an actuation portion of the fitting portion 4a, there are incorporated a thermometer for measuring the internal head, headphones that allow listening to music, glasses for shining colored light on the eyes and a vibrator for stimulating the ears' acupuncture spots.

The thermometer 5 for measuring the internal body temperature measures the rectum temperature with a sensor 11 inserted from the anus. The measured value of this thermometer is displayed on the control panel that is not shown. Therefore, the patient continues warm bathing for the duration of an optimum treatment time after this measured value reaches the thermal sensitivity temperature band matching with the cancer type of the patient. The temperature of the warm source is regulated by the heating apparatus so as to maintain the patient's internal body temperature within the thermal sensitivity temperature band.

In the portion of the bed 7 touched by the patient, a magnetic field inducer 12 is attached to improve the magnetic field (biological magnetic field) occurring therearound as a result of flow of biological current. The magnetic field inducer 12 is formed with two or more kinds of jewels or metals in powder, granule, lump or plate form.

The efficacies of jewels forming the magnetic field inducer 12 are as shown in Table 7. For the purpose of resolving the patient's stress, jade, hematite, amethyst, carnelian, garnet, tiger eye and turquoise are, for example, probably preferred.

Use of metals rather than jewels provides a metal ion generator, allowing freed negative metal ions to penetrate into the body through the skin and showing improvement in biological current as with jewels.

The jewels and metals may be used in combination. Alternatively, the magnetic field inducer 12 may be configured such that jewels or metals come in contact with the skin (for example, attached on the inner side of a ring) so that the patient can wear the magnetic field inducer 12.

Thanks to the aforementioned functions of jewels/metals, bodily cells are activated, and stress resolving effect is obtained.

The bed 7 is split into a leg portion 7a and a rear portion 7b, with the angle between the leg and rear portions being adjustable and the height also being adjustable.

When non-convective mud or wood dust is filled into the bathtub as a warm source, a heater is attached to the bathtub as the heating apparatus 9.

In the case of hyperthermia using the apparatus configured as described above, a warm source 13 (preferably mud) is filled into the bathtub 1. While a liquid such as water may be acceptable as the warm source, mud - a substance with a high specific heat - is preferred. After the warm source 13 is heated, the patient is put in the bathtub 1 and placed on the bed.

Next, the thermometer tube 10, a mask of the anesthesia apparatus, the hat-shaped actuation portion 4a and the sensor 11 of the internal body temperature measuring instrument are attached to the patient, and the switches thereof are switched on.

Once the switches of the apparatuss are switched on, the head is cooled by the hat-shaped actuation portion 4a, with a colored light having a relaxing effect entering the eyes, a sound (music) having a relaxing effect entering the ears from the headphones, anesthesia gas supplied from the anesthesia apparatus and the ears' acupuncture spots stimulated by the vibrator. Concurrently, bodily current is put in order by the action of the magnetic field inducer 12 installed on the bed 7.

Thanks to such actions, the patient's brain stress is gradually resolved although treatment is still underway. Concurrently, the internal body temperature rises by the action of the warm source. Since change in internal body temperature can be grasped by the internal body temperature measuring instrument 5, the patient is continuously subjected to warm bathing for the duration of optimum treatment time after the internal body temperature reaches the thermal sensitivity temperature band.

By using such a procedure, it is possible for the patient to receive hyperthermia while keeping patient stress to a minimum.

Findings from administration of the warm bathing treatment of the present invention to cats contracted with FIV, show that all three specimens tested FIV negative.

FIV and HIV are considerably alike in gene sequence, and it is said in medical circles that if it is possible to completely cure FIV, it is highly likely that HIV will also be completely cured. Cats' body temperature is similar to the humans' or 36.5 to 37°C.

The conditions of the aforementioned test are as follows:
Warm source type Light-blue warm water
Temperature and warm bathing time 42°C, 2 hours, bathtub covered
Other stress relaxing means

### Injection of anesthesia gas-oxygen mixture

### Head cooled

Two or more kinds of metals and two or more kinds of jewels were brought in contact with the specimens.

### INDUSTRIAL APPLICABILITY

According to the present invention, it is possible to administer hyperthermia in such a way to reduce stress to the extent possible. It is also possible for the patient to receive treatment in a way almost similar to normal bathing, relieving the patient from pain attendant on treatment.

Moreover, while it was impossible to irradiate the whole-body simultaneously with conventional hyperthermias using, for example, far infrared ray, heat acts on the whole-body from all directions according to the present invention, penetrating thereinto. Water and other warm sources have a high specific heat (water is 26-fold higher than air), making it possible to raise the deep body temperature by wrapping the body with temperatures, 40 to 45°C, that are in no way likely to cause burn and allowing warm heat to uniformly penetrate the whole-body.

Conventional hyperthermias using far infrared ray and so on require temperatures of 60°C to 78°C, making the treatment unbearable unless the patient is put under constant anesthesia. Besides, there was a concern over adverse impact of anesthesia to the cranial nervous system. According to the present invention, however, it is possible to raise the internal body temperature beyond 40°C using a warm source at 40°C to 45°C, thus allowing administration of hyperthermia with a restricted supply of anesthesia gas while at the same time supplying a sufficient amount of oxygen. This makes it therefore possible to prevent adverse impact to brain cells to the extent possible.

Moreover, catheter puncture/insertion, a dangerous conduct often used in conventional hyperthermias, is completely eliminated, with no electromagnetic wave irradiation, a conduct that has not been safety tested.

It is, therefore, possible according to the present invention to administer hyperthermia safely with pain to the patient reduced to the extent possible.

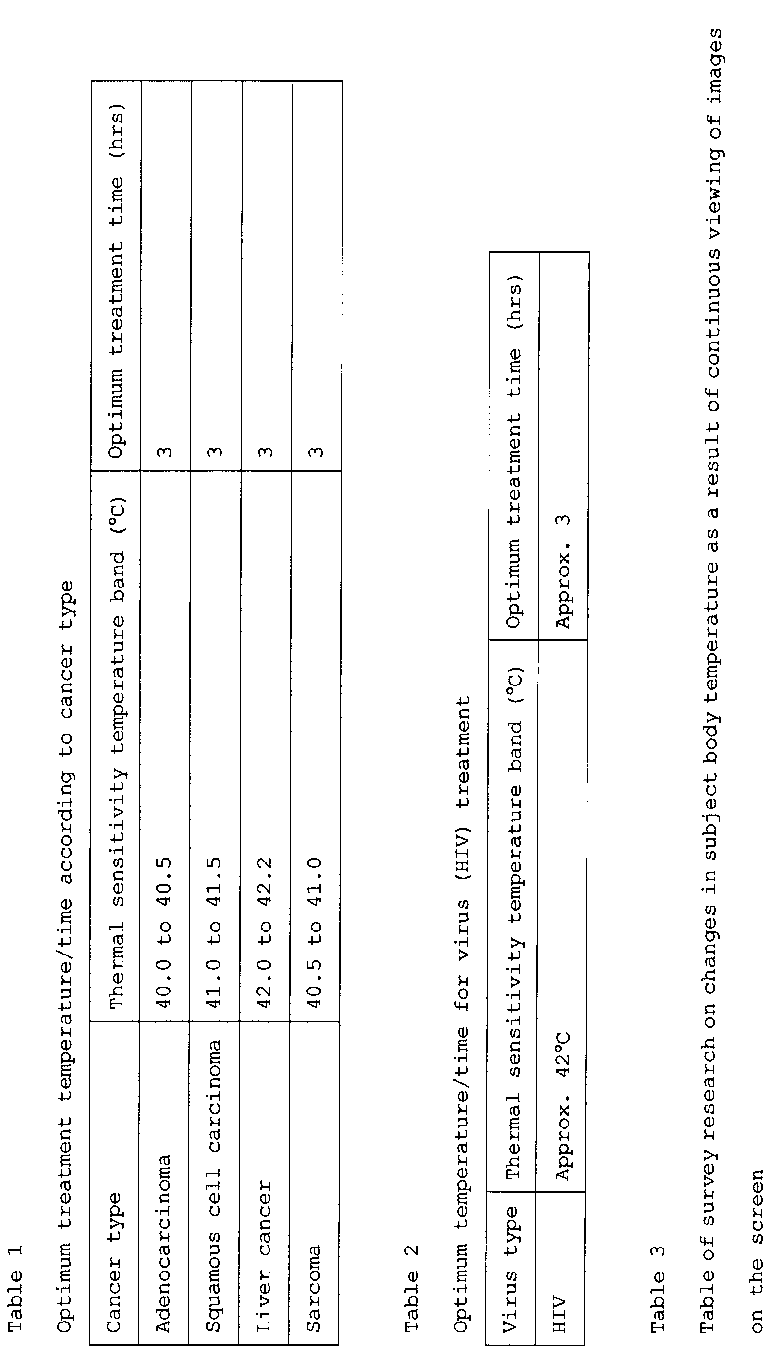

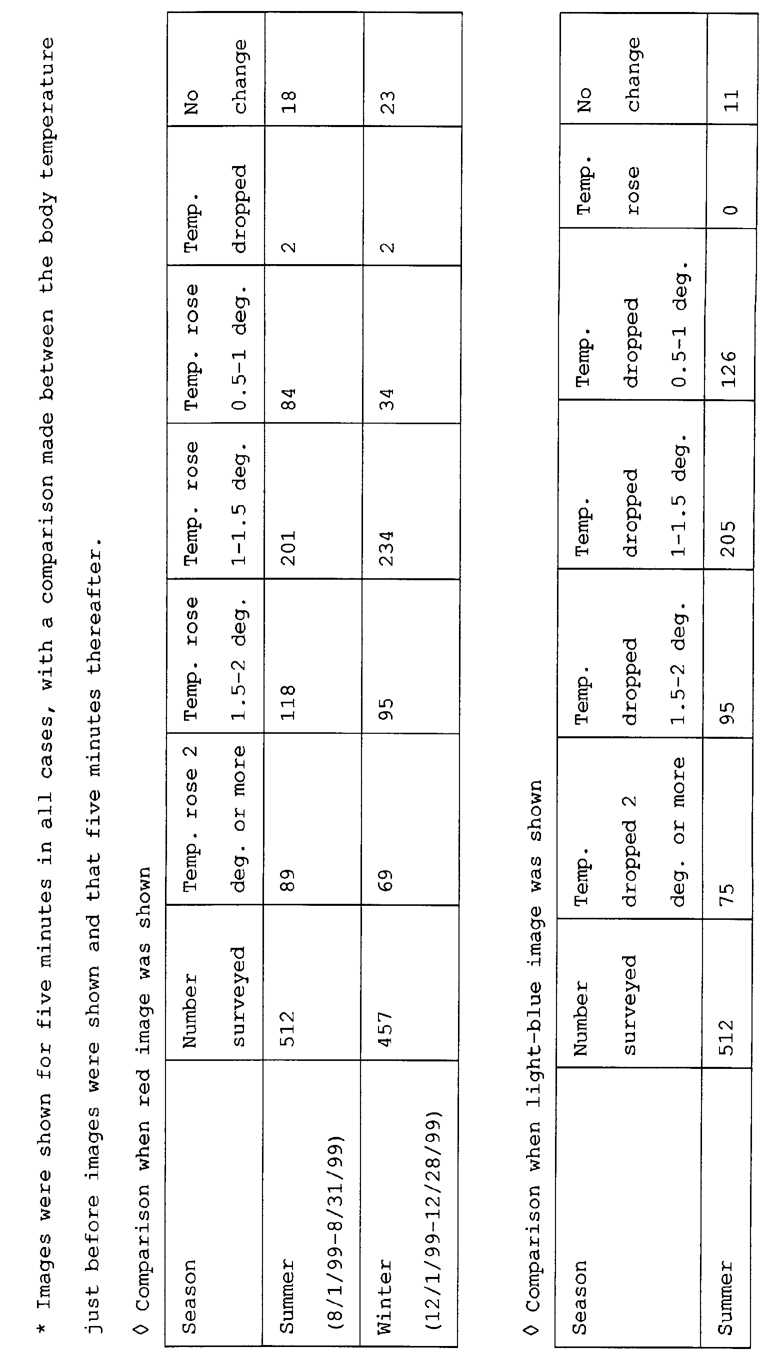

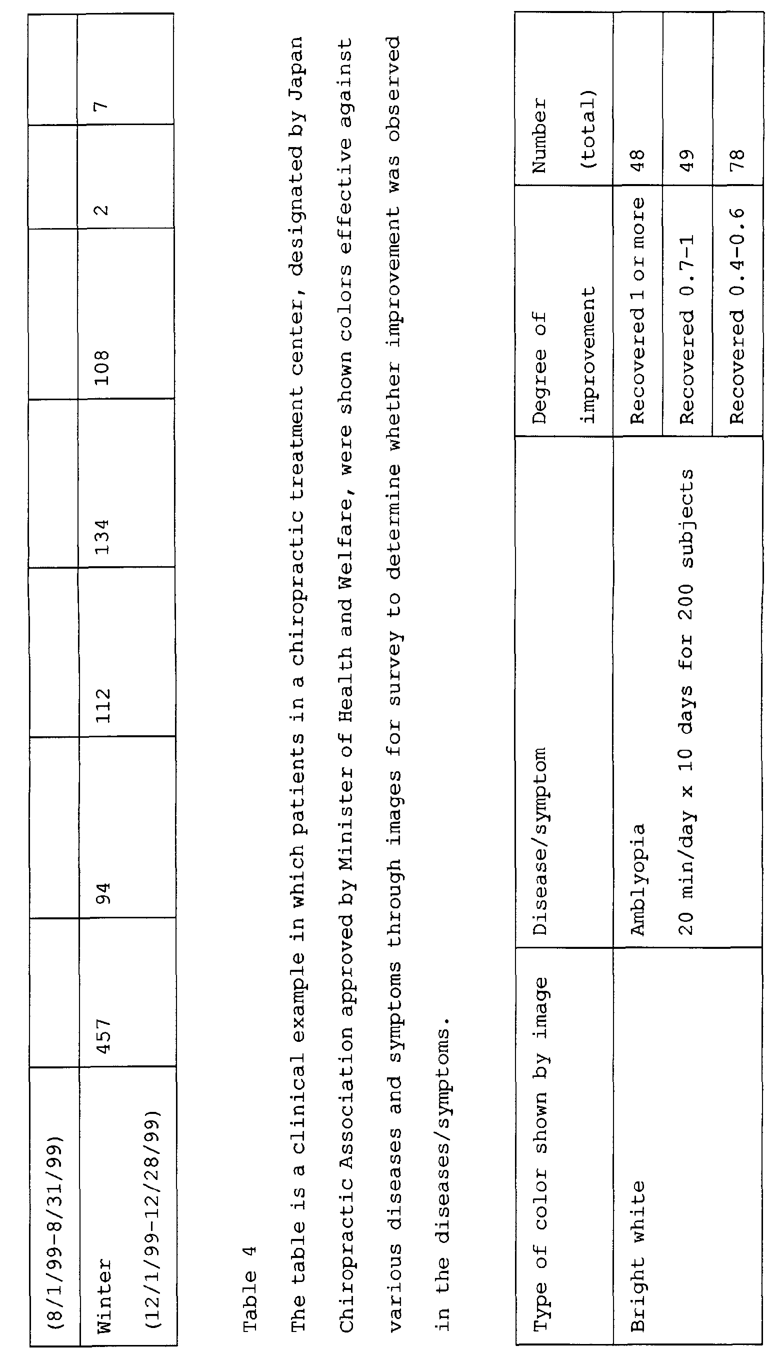

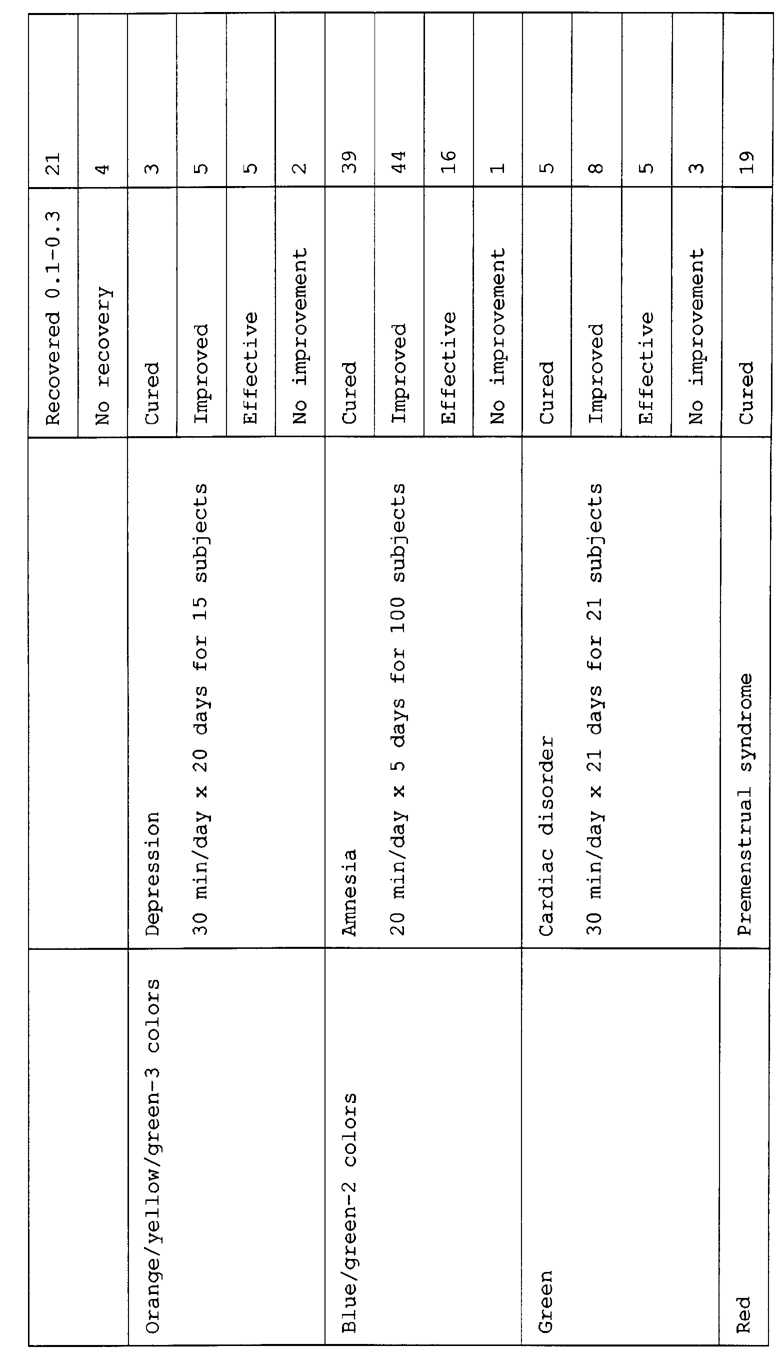

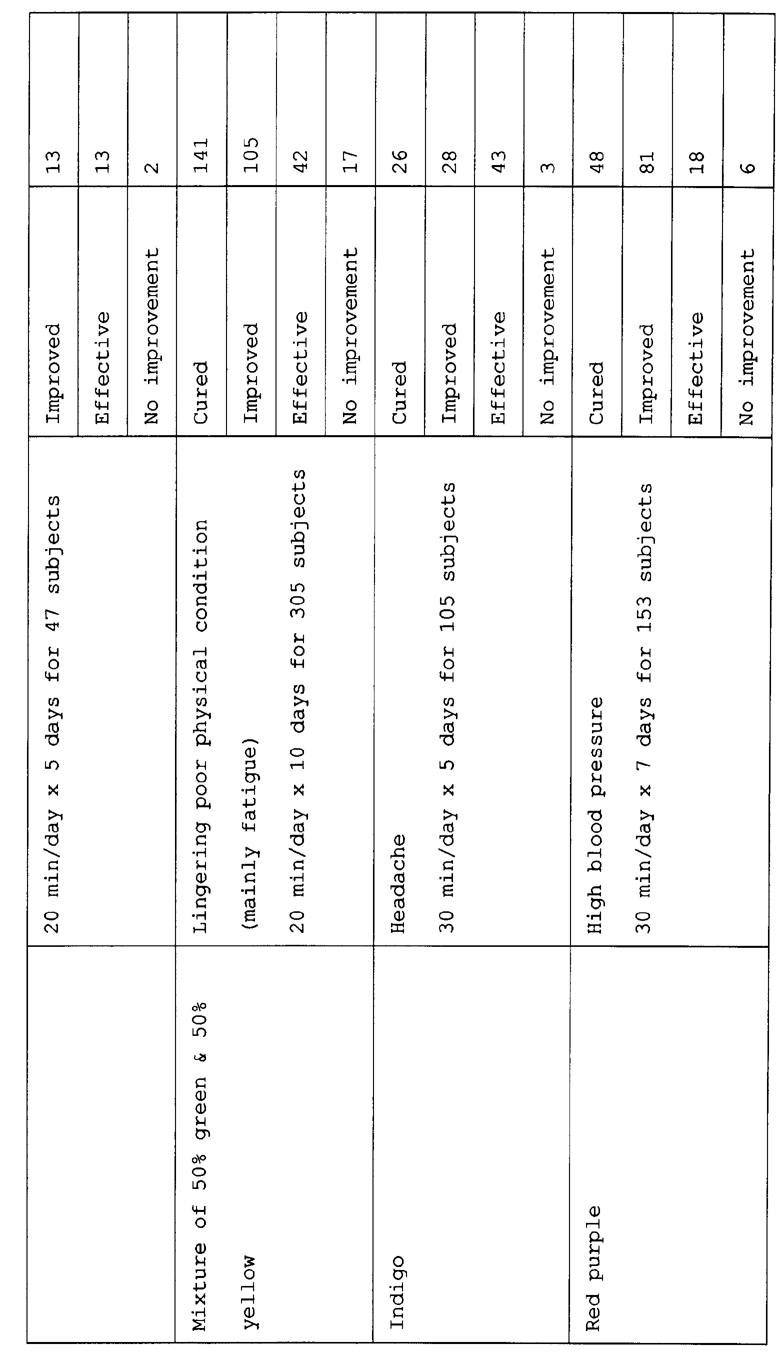

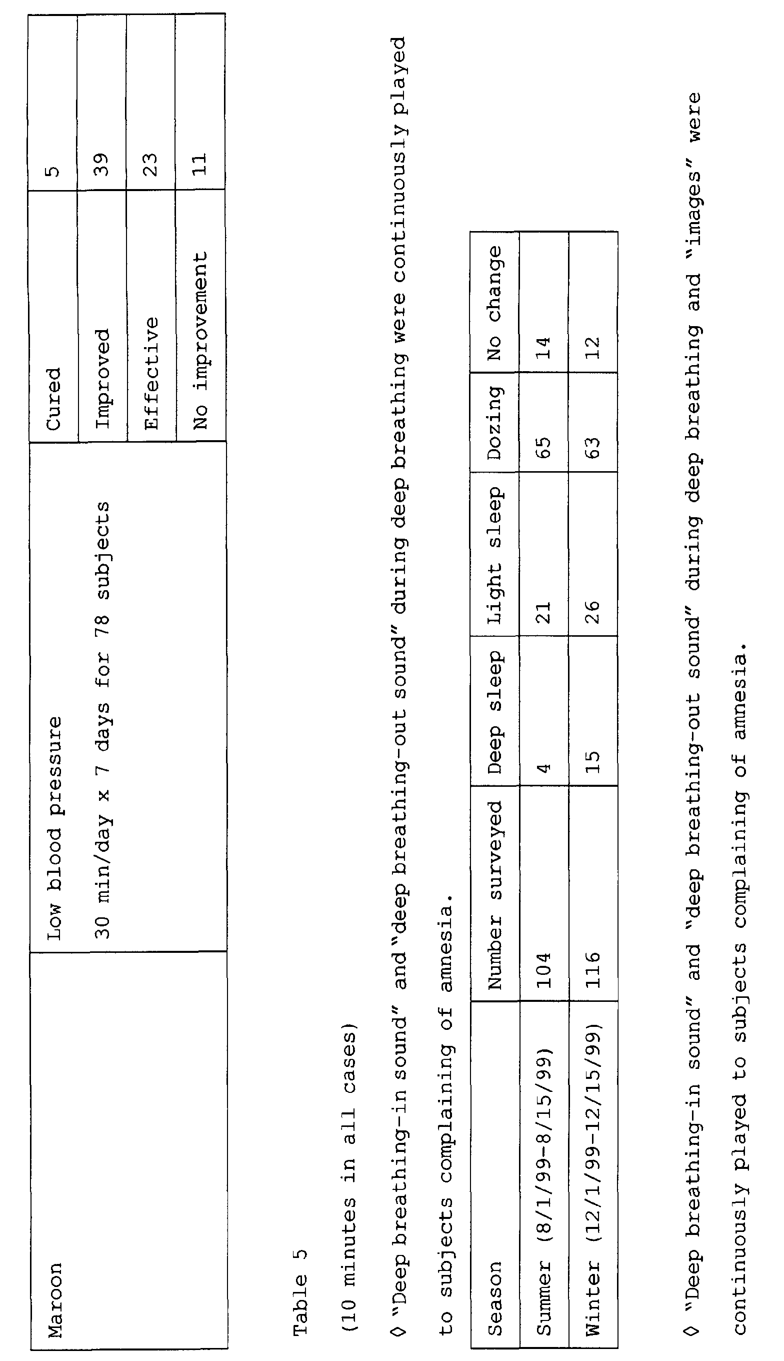

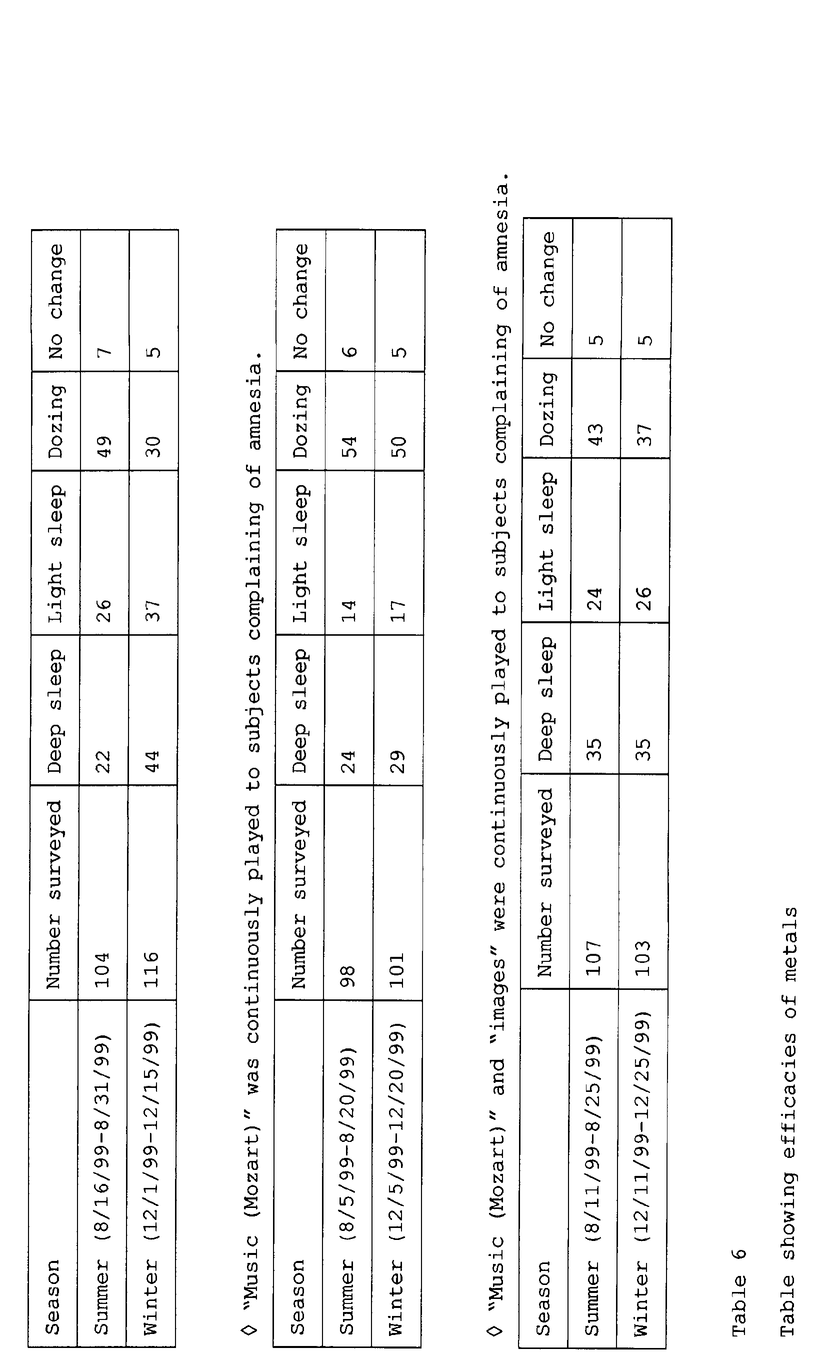

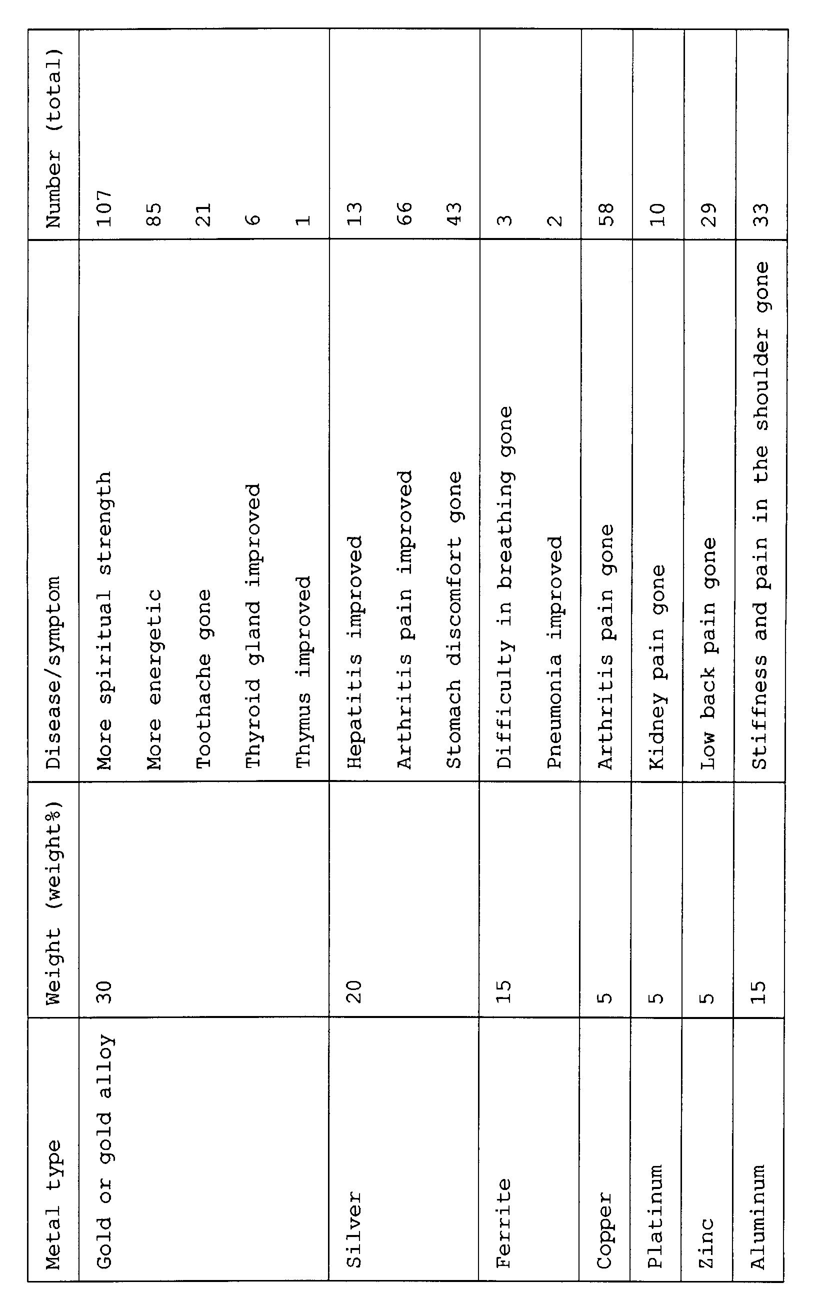

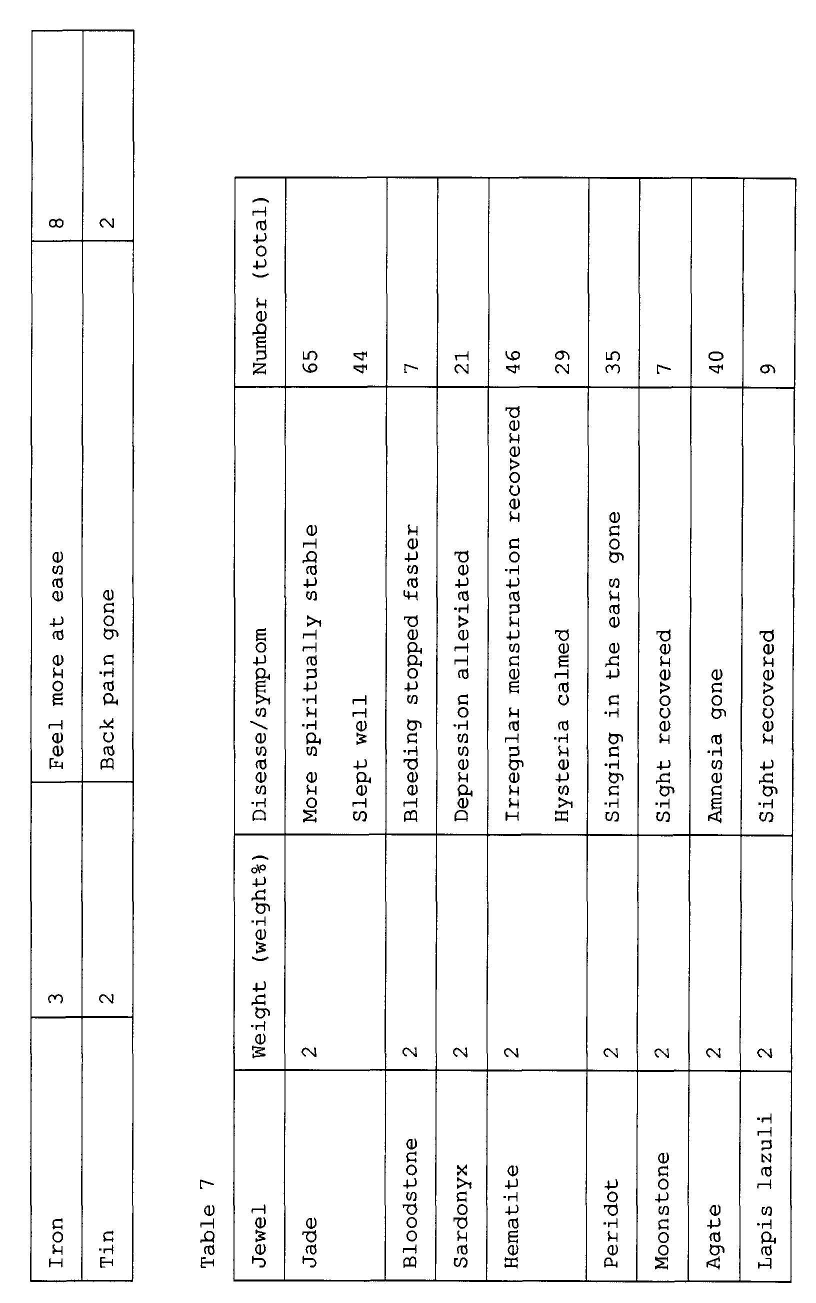

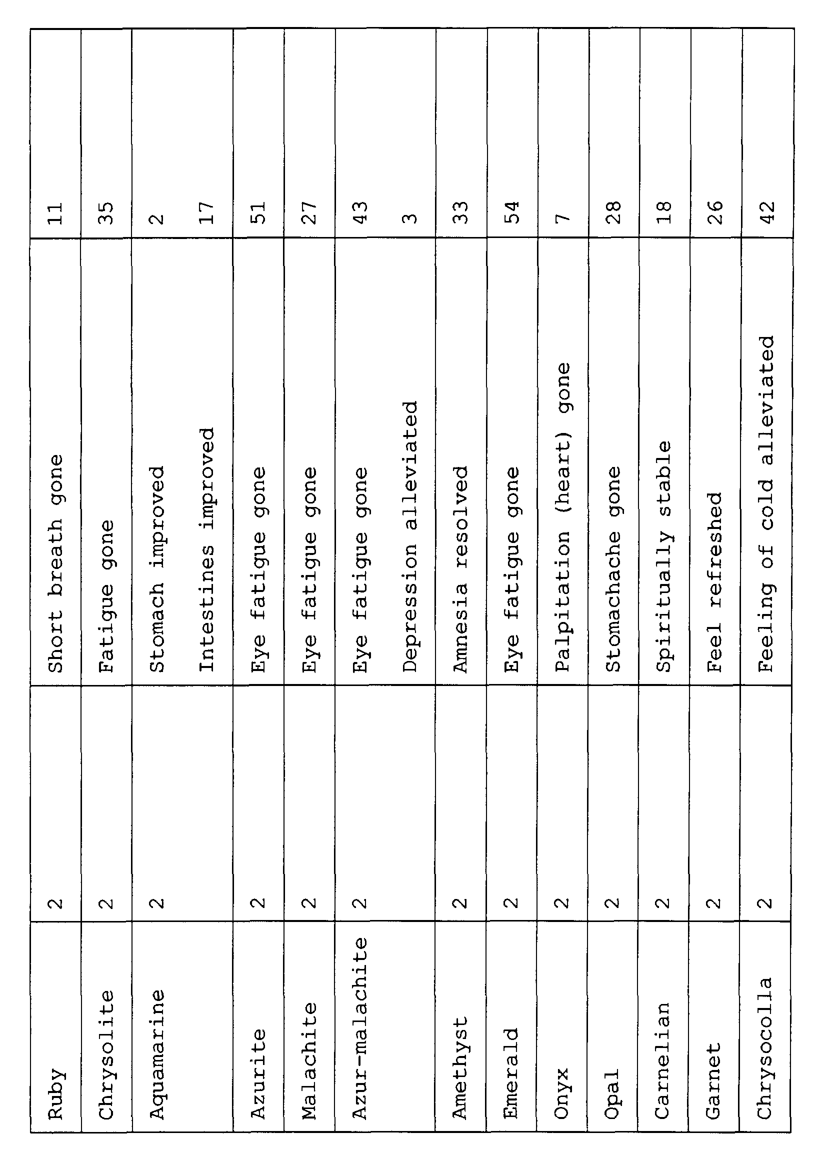

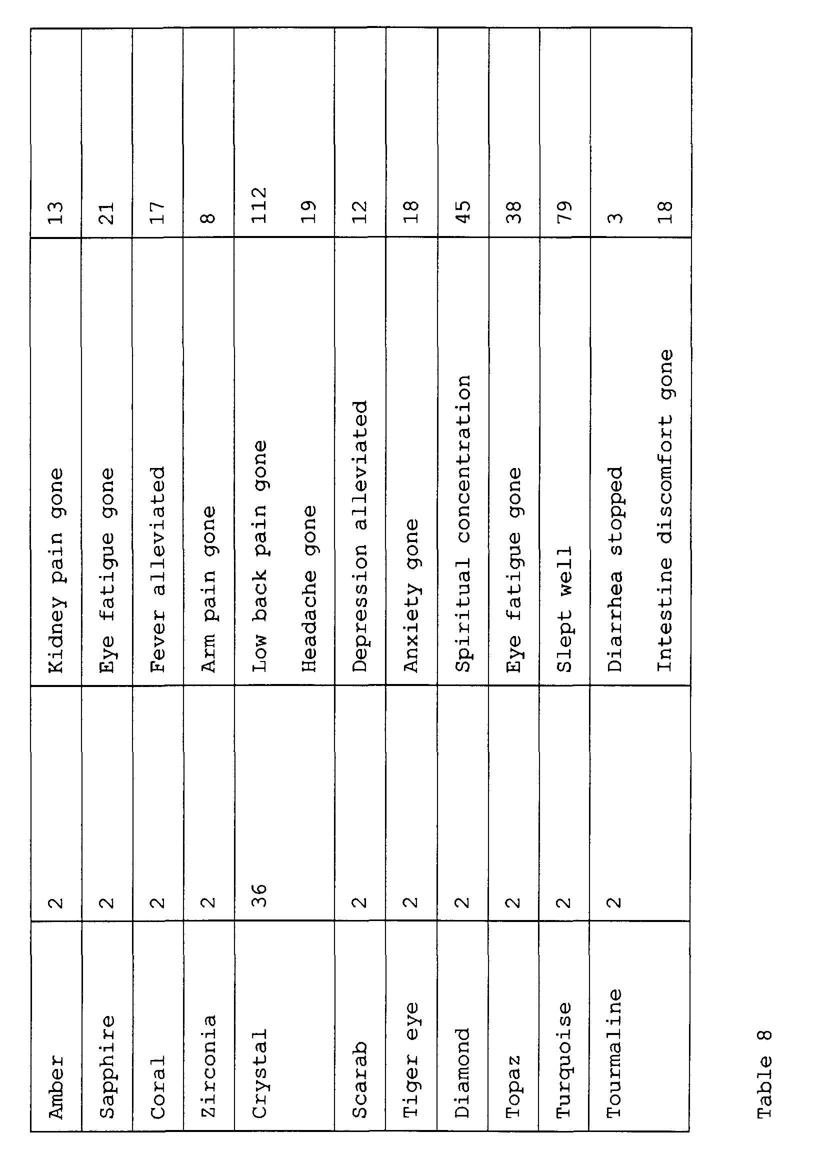

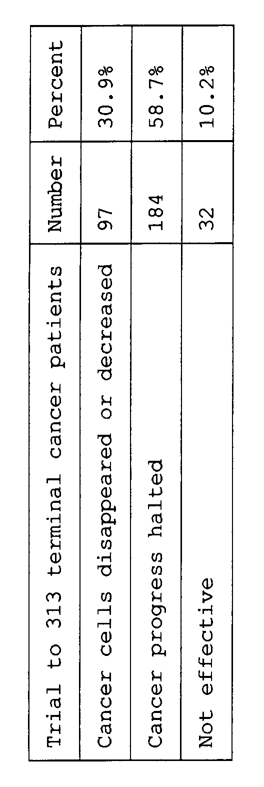

## Claims

1. A whole-body hyperthermia, wherein a patient is subjected to warm bathing while at the same time administering treatment to minimize brain stress and measuring the internal body temperature.

2. The whole-body hyperthermia of claim 1, wherein
the treatment to minimize brain stress includes any one or a combination of two or more of the following:
(1) using water colored in blue, light-blue or other color as a warm source for warm bathing;
(2) shining colored light on the eyes;
(3) allowing the patient to listen to sounds;
(4) stimulating shiatsu or acupuncture spots;
(5) cooling the head; and
(6) resolving brain stress, by the action of metal ions and magnetic field, by attaching a biological current adjustor, consisting of two or more metals or jewels of different kinds, to the patient or to a portion of the bed coming in contact with the patient.

3. The whole-body hyperthermia of claim 1, wherein the warm source for warm bathing is water, mud or any other substance having a specific heat higher than that of water.

4. The whole-body hyperthermia of any one of claims 1 to 3, wherein the patient drinks, during warm bathing, any one or a combination of two or more of water, aqueous trace element solution and aqueous solution in which trace elements are copied to potassium iodide water.

5. A whole-body hyperthermia apparatus comprising:
means for measuring the internal head temperature of the patient;
means for measuring the internal body temperature of the patient; and
means for resolving brain cell stress of the patient.

6. The whole-body hyperthermia apparatus of claim 4, wherein the means for resolving brain stress includes one or a combination of two or more of the following:
(1) means for shining colored light on the eyes;
(2) means for allowing the patient to listen to sounds;
(3) means for stimulating shiatsu or acupuncture spots;
(4) means for cooling the head; and
(5) a biological current adjustor, consisting of two or more metals or jewels of different kinds, attached to a bed fitted to a bathtub.
